# EUROPEAN PATENT APPLICATION

(11) **EP 4 663 278 A2**
(43) Date of publication of application: **17.12.2025**
(21) Application number: 25181574.2
(22) Date of filing: 09.06.2025
(51) Int. Cl.: B01D 61/44

(54) **METHOD OF CONDUCTING ELECTRODIALYSIS AND METHODS OF OBTAINING LACTIC ACID USING THIS ELECTRODIALYSIS**

(30) Priority: 14.06.2024 PL 44883724
(71) Applicant: Orlen Poludnie Spolka Akcyjna, 32-540 Trzebinia (PL)
(72) Inventor: Pajor, Aleksandra, 32-555 Zagorze (PL); Wieclawik, Justyna, 42-260 Rudnik Wielki (PL); Szkarlat, Anna, 30-012 Krakow (PL); Ziembinski, Szymon, 32-540 Trzebinia (PL); Szeligowski, Adam, 30-383 Krakow (PL); Semerjak, Grzegorz, 32-500 Chrzanow (PL); Jemiolo, Bartlomiej, 32-608 Osiek (PL); Koziarz, Karolina, 43-600 Jaworzno (PL); Trzaskowska, Wanda, 30-014 Krakow (PL); Bialas, Wojciech, 62-030 Lubo (PL)
(74) Representative: Kondrat, Mariusz

(57) **Abstract**

The object of the invention is a method of conducting electrodialysis suitable for the conversion of sodium lactate into lactic acid, characterised in that into a three-chamber electrodialyzer with a bipolar module made of cell pairs with a BPM-AM-CM membrane arrangement, a feed which is a solution of sodium lactate with a concentration of 50 to 200 g/L of sodium lactate is introduced, wherein three products are obtained as a result of the method: a lactic acid solution, a sodium hydroxide solution and a depleted superfluid stripped of most of the sodium and lactate ions, and wherein the cathode and anode electrode solution is an aqueous sodium hydroxide solution. The object of the invention are methods of obtaining lactic acid using this electrodialysis process.

## Description

### TECHNICAL FIELD

The object of the invention is a method of obtaining and purifying and separating lactic acid from actual fermentation broth, in which, among other things, molasses is used as the sole carbon source. The process according to the invention is carried out in an innovative manner using the process of adsorption of colour compounds on activated carbon and conversion using the three-chamber bipolar electrodialysis technique.

### BACKGROUND ART

Lactic acid, with the molecular formula CH₃CH(OH)COOH, is an organic carboxylic acid produced mainly by microbial fermentation of carbohydrates. A much less commonly used alternative is to obtain lactic acid through chemical synthesis processes [1]. In both the biotechnological pathway for acid production and the chemical production route, the challenge is to develop a process that results in a high-purity product, while maintaining the profitability of the process and environmental neutrality. These important aspects characterising lactic acid production technology are mainly influenced by the raw materials used (their purity, cost, share in the culture medium), the by-products generated (their potential for reuse in the process), the waste generated (for example, gypsum, organic and inorganic solvents), the number of operations used, their energy consumption and the complexity of the process.

Among the methods described in the state of the art of obtaining lactic acid, the biotechnological method is predominant. In this process, the most commonly used microorganisms are *lactic* acid bacteria (LAB, or *Lactic Acid Bacteria)* [2]. Fermentation takes place in a bioreactor under strict process conditions in a sterile liquid culture medium. In this process, a number of interrelated biochemical and physical processes take place, such as anabolic processes (synthesis of complex compounds from simple substances - cell growth, production of bioproducts), catabolic processes (breakdown of complex compounds into simpler compounds with the release of energy - consumption of carbohydrates), and physicochemical processes (changes in viscosity, pH). The result of microbial metabolism is an extracellularly produced product, which is neutralised by a dosed alkali during bioprocessing. This is a well-known and frequently used practice that enables the process to be run optimally and reduces the negative effects of too low pH values on the bacteria. However, the details of this operation may vary depending on the microbial strain used. The process thus carried out results in a product in the form of a lactic acid salt, the form of which is determined by the type of base dosed. In the subsequent steps, the product must be separated and purified. The state of the art describes various substrates for conducting fermentation processes to obtain lactic acid salts.

Application US2013266995A1 discloses a method of producing D-lactic acid from sugar fermentation using a carbon bed. In the described solution, a sterile activated carbon rod is introduced into a fermentation bioreactor where microorganisms produce lactic acid, and adsorbs the lactic acid. Lactic acid is recovered from the bed by using acetone. The use of significant amounts of this organic solvent contradicts eco-design and does not make the technology industrially attractive, especially as the authors do not disclose the possibility of multiple use of this organic medium (recirculation). The viability of the carbon rods used is also not disclosed. Furthermore, the fermentation medium used is based on pure glucose (monosaccharide in their pure form are a very easily digestible carbon source for bacteria) and contains the addition of yeast extract and tryptone. This composition of culture medium is not optimal from an economic point of view.

Application WO/2004/057008 describes a solution in which the main (but not the only) sugar source in the culture medium is beet molasses. In the solution presented, the authors disclose the need to pretreatment the molasses by dilution, acidification with sulphuric acid(VI), boiling, centrifugation and filtration. The complex pretreatment process adversely affects the energy efficiency of the process and significantly increases its cost.

In document EP2609989A1, fermentation is carried out by adding one or more proteolytic enzymes to the fermenter to achieve continuous production of hydrolysed protein with simultaneous fermentation by a lactic acid-producing bacterial culture.

In document US2011210001A1, the inventors use products of agro-food origin. Their technology includes an additional step in the form of pretreatment of raw materials (grinding, filtration). In addition, an enzyme preparation is added to the prepared culture medium, which hydrolyses the sugars and facilitates their uptake by the microorganisms. The complex method of preparing the raw material and the nutrient medium, together with the dosing of the enzyme preparation, makes the process multi-stage and potentially costly.

The physical, physico-chemical and chemical pretreatment of raw materials of natural origin is very effective, but costly due to high energy consumption. In technologies that involve pretreatment of materials, the chemicals used can produce streams with high contamination potential and generate nuisance by-products that cannot be reused at all or without additional treatment steps [2].

Beet molasses, obtained as a by-product of the sugar production process, is rich in sucrose (share of approximately 50%), which makes it a desirable substrate for fermentation processes. However, it carries a number of colouring substances, mainly caramel and melanoidin compounds, which can affect the colour of the final product obtained after the lactic acid fermentation process.

Once the lactate salt is obtained, a series of technological processes take place to purify the lactate salt and convert it to lactic acid.

Patent EP2609989A1 describes a method for producing lactic acid from fermentation broth that contains sodium lactate. The start of the purification process involves centrifugation of the fermentation broth to remove suspended solids and fibres, thereby generating a stream of clarified fermentation broth and wet biomass combined with fibres. The disclosure highlights that the centrifugation process, when applied prior to operations such as membrane microfiltration, reduces the frequency of backwashing and increases permeate flow. Additionally, the authors disclose that the wet sludge produced by the centrifugation process contains between 5% and 25% of the fermentation broth. Therefore, in order to reduce the loss of lactate, a centrifugal decanter was used to obtain the dry matter from 0.5% to 2.5% of the residual fermentation broth. The centrifugation process alone therefore generates significant product losses. The additional decanting process used reduces losses, but generates the need for an additional operation, which can increase process costs and duration.

One of the processes used to separate lactic acid from its salts is electrodialysis. Electrodialysis involves the directed transport of ions across membranes under the influence of an applied constant electric field. For current techniques, the choice of electrodialytic stack configuration is crucial. For electrolyte concentration, anion-selective and cation-selective membranes should be used (classical electrodialysis), while the conversion of salt to acid and base can be carried out using bipolar membranes (bipolar electrodialysis). The choice of membrane material and system depends on the efficiency and effectiveness of the process.

Electrodialysis processes use ion-selective membranes (anion-exchange, cation-exchange, amphoteric, bipolar), whose separating capacity is determined by the type of functional groups built into the hydrophobic matrix, usually made of cross-linked polystyrene, polyethylene or polysulfone. The type and concentration of functional groups determines the mechanical properties, permeability, as well as the conductivity and electrical resistance of the membranes.

The effectiveness and efficiency of electrodialysis can be limited by certain phenomena associated with current processes, i.e. backward diffusion, osmosis, electroosmosis or electromigration. The course and efficiency of electrodialysis are also negatively affected by phenomena such as ion migration, the occurrence of *fouling* or the possibility of membrane poisoning.

Despite the many difficulties associated with the application of the electrodialysis process as a conversion and separation technique, the state of the art indicates that both classical and bipolar electrodialysis can effectively purify and concentrate low-molecular-weight compounds from the fermentation broth. Most often, a series of operations are performed prior to the electrodialysis step to adapt the feed to the current process.

Patent EP1618202A1 (WO2004/057008 A1) discloses a typical method of converting sodium lactate to lactic acid by means of bipolar electrodialysis with a two-chamber module, which can be inferred from the description of the two streams obtained in the process - one containing lactic acid and residual sodium lactate and the other containing sodium hydroxide solution. According to the solution, the extracted NaOH solution is used directly to adjust the pH in the fermentation process. Example applications of the disclosed technology detail membrane filtration, ultrafiltration and nanofiltration steps, a column system with ion exchange resins and chelating agents, which precede electrodialysis carried out in two steps, which also leads to the concentration of the resulting lactic acid solution. However, the adaptation of two successive electrodialysis steps, instead of one, undoubtedly generates an increase in the electrical energy required to carry out the two-step electrodialysis and indicates an insufficient primary concentration of lactate salts in the working solution being processed, since it requires an additional concentration step by electrodialysis.

Patent EP2534252B1 describes conducting electrodialysis of an aqueous solution of a monovalent lactate salt with a concentration of 10-30%, preferably 20-25%. Prior to the electrodialysis step, the lactate solution can be used directly if it has the required concentration, can be diluted to a given concentration range or can be concentrated by distillation or an additional electrodialysis step. According to the description of the technology, electrodialysis is characterised by a conversion rate of 40 to 98% mol, preferably 85% mol. According to the description, the electrodialysis process is carried out in a conventional two-chamber system, which is due to the two streams generated in the process: a solution of a monovalent base and a solution of lactic acid and unconverted lactate salt, and the construction of a stack of cation exchange membranes and bipolar membranes. The area of the membranes is 0.01m², operating at a constant current of 7.5 A. In the example described, 2% sulphuric acid in aqueous solution was used as the electrode solution for the anode, the electrode solution for the cathode was 8% sodium hydroxide solution and the working solution for the feed contained 20% sodium lactate. In the presented solution, according to the authors of the disclosure, the electrodialysis process carried out at an elevated temperature exceeding 50°C, and preferably in the range of 60-80°C, would translate into reduced energy consumption. According to common knowledge, the bipolar electrodialysis process is typically carried out in the range of 25-40°C and up to 60°C at most, which is a result of the limited life of the membrane components of the electrodialytic stack, which are usually not resistant to high temperatures. The electrodialysis system components presented in the example can operate at a maximum operating temperature of up to 40°C (the cation exchange membrane up to and including 60°C and the bipolar membrane up to and including 40°C) according to the manufacturer's specifications, although the authors typify an operating range between 4 and 60°C. Consequently, the electrodialysis step itself is carried out in a typical manner for this type of process if the membranes have not been expertly modified to ensure operation at higher operating temperatures than those predicted by the manufacturer, which is not disclosed in the invention description.

The electrodialysis step mentioned in the claims of patent WO23006876 A1 refers only to the execution of the process in a two-chamber system with a cation exchange membrane and a bipolar membrane. The description of the technology mentions the general possibility of performing the bipolar electrodialysis process in a three-chamber system as a known process solution, but according to the content of the patent and its claims the solution is not part of the patented technology. In addition, only a conventional technological solution for performing electrodialysis is indicated as the form of process implementation in the examples described. The surface area of the membranes is only 0.01m², and the working solutions are the anode electrode solution in the form of 2% sulphuric acid, the cathode electrode solution being 8% sodium hydroxide solution, the working solution of the feed as 20% sodium lactate solution. The process temperature was 40-60°C. The process was carried out at a constant current of 7.5 A and a stack voltage of 9.5 to 22.4 V. As an effect of the invention according to WO23006876 A1, lower energy consumption was indicated when implementing an electrodialysis process using potassium lactate compared to sodium lactate. The disclosed technology reserves the production of lactate in the form of a magnesium salt, which is successively converted to a monovalent lactate salt, resulting in a by-product in the form of solid magnesium hydroxide, which requires additional separation from the solution of the monovalent lactate salt. The further described technology requires additional concentration of the aqueous lactate salt solution to a concentration of 30-45 %. According to the disclosure, the preferred concentration step comprises the separation of water from the aqueous lactate salt solution. This constitutes an additional unit operation, which, in the case of industrial-scale implementation of the technology and processing of large volumes, generates apparatus, energy and material costs and adversely affects the operating times of the entire technology. Subsequently, the concentrated stream of lactate is directed to the step of bipolar electrodialysis conducted in a two-chamber system. Moreover, according to the authors' description, the presented data and the developed electrodialysis and distillation solutions were obtained for solutions representing a model of process mixtures that could come from fermentation, prepared from standard two pure substances such as lactic acid and food-grade sodium lactate. This type of approach does not guarantee the described efficiency of the individual technology steps using problematic and multi-component real mixtures obtained from the fermentation of substrates of natural and/or waste origin.

Lactic acid, its salts and esters find a great many applications in the food, chemical, cosmetic and pharmaceutical industries. As a food additive, lactic acid is a good stabiliser and preservative, added for example to sauerkraut, olives and pickled vegetables. It is used as an acidifier, flavouring agent, pH buffer or as a bacterial growth inhibitor in a wide range of processed foods such as confectionery, bread and bakery products, soft drinks, soups, sorbets, dairy products, beer, jams and jellies. Due to its properties, lactic acid is gaining popularity as a precursor to biocompatible and biodegradable materials. In particular, we are referring to the polylactic acid (PLA) polymer, which is becoming a viable alternative to commonly used petrochemical plastics. PLA has begun to be successfully used as a substance for packaging in the form of films, coatings and semi-rigid packaging, as well as for prosthetics, sutures and controlled-release carriers in the medical, pharmaceutical and textile industries. The increasing demand for PLA generates the need to prepare high-purity lactic acid as a raw material for polymer production. Depending on the production process used, lactic acid exists in the form of one of the enantiomers D(-) or L(+), as well as in the form of a racemic mixture. In order to obtain the desired high quality PLA, it is necessary to provide optically and chemically pure lactic acid. The individual pure isomers are not obtained by chemical synthesis, they are only obtained by microbial fermentation. Consequently, lactic fermentation has for years been a commercial means of obtaining lactic acid as a monomer for the production of polylactide. The industrial processes described are based on homofermentation carried out by selected strains of lactic acid bacteria. It is the metabolism of the microorganisms that determines the production of an optically pure product. In addition, chemical purity must also be taken care of. Although the process of producing lactic acid by fermentation has been known for many years and has been well understood, the **complex separation and purification procedure** is still the main challenge in the commercial production of this compound as a raw material for further polymerisation.

### PURPOSE OF THE INVENTION AND THE TECHNICAL PROBLEM

Due to the growing interest in the use of lactic acid in industry, there is a need to develop new high-performance processes leading to a **high-purity product with reduced energy requirements.** This is particularly relevant today, when the finite earth's reserves of oil, coal, natural gas, geopolitical problems and the associated rising energy prices are an obstacle to free operation of large-scale technological processes. In addition, in view of the pollution of the planet, the development of technologies that minimise energy demand is part of pro-environmental measures. It is observed that in the total energy demand of the entire lactic acid production technology, the energy consumption of the electrodialysis process accounts for a significant share, and as is known, this consumption is closely linked to:
- quality of the feed (concentration of lactate salt, purity of the feed in terms of fermentation residues),
- the type of electrodialysis carried out,
- the type and arrangement of membranes in the electrodialytic device.

The inventors of the invention have observed that conducting electrodialysis under certain conditions results in the process showing lower energy consumption, while maintaining good product yield and purity. The effect achieved for the electrodialysis step translates into the productivity and energy efficiency of the whole technology of obtaining lactic acid.

In addition, it was observed that carrying out the adsorption process, especially of coloured compounds, on activated carbon leads to a significant reduction of coloured compounds, most of which are derived from molasses. The process of adsorption of coloured compounds on activated carbon allows the preparation of the feed in such a method that the subsequent electrodialysis process runs smoothly. This shows that there is a relationship between the adsorption process on activated carbon and the electrodialysis process.

The electrodialysis process carried out in the inventive manner also allows for a reduction in the amount of auxiliary media used in the technology. Surprisingly, it was found that the sodium hydroxide solution generated in the electrodialysis process can be used as the electrode solution for both the anode and cathode. This avoids the use of additional means in the form of, for example, the commonly used sodium sulphuric acid and sodium sulphate solution, as well as the supply of sodium hydroxide from external sources.

The present invention is in the trend of pro-ecological solutions, taking into account the circulation and valorization of the by-product stream, which becomes the feed stream. The technical problem that the invention solves is to **reduce the energy consumption needed to run the key process of the lactic acid production technology - electrodialysis,** while maintaining good productivity and purity of the lactic acid.

Another technical problem that the invention solves is the **reduction in the amount of additional auxiliaries introduced into the technology,** which could be achieved as a consequence of the new conditions used in the electrodialysis process. Specifically, the sodium hydroxide solution formed in the process was used as the electrode solution for both the cathode and the anode.

The use of an activated carbon adsorption process with inventive parameters, on the other hand, solves the problem of **limited capacity in the processing of a difficult raw material** such as molasses. Thus, the technology manages a by-product from the food industry and is based on a readily available renewable raw material, the processing of which does not adversely affect the environment. Colour compounds, whose presence in the final product is undesirable, are removed prior to the electrodialysis process, while retaining a surprisingly low retention rate of lactate ions. All this has a positive effect on the electrodialysis process, both on membrane durability and, in a non-obvious method, on improving the efficiency of the process, while maintaining good lactic acid yield and purity. Delivering an optimum concentration of sodium lactate directly to the electrodialysis node results in a reduced energy intensity of the process. The synergistic combination of the electrodialysis process according to the invention and adsorption on activated carbon according to the invention makes it possible to achieve a lactic acid solution with high chemical purity.

### ESSENCE OF THE INVENTION

The invention addresses the above-mentioned problems.

The object of the invention is a method of conducting electrodialysis suitable for the conversion of sodium lactate into lactic acid, characterised in that into a three-chamber electrodialyzer with a bipolar module made of cells with a BPM-AM-CM membrane arrangement, a feed which is a solution of sodium lactate with a concentration of 50 to 200 g/L of sodium lactate is introduced, whereby three products are obtained as a result of the method: A lactic acid solution, a sodium hydroxide solution and a depleted filtrate stream devoid of most sodium and lactate ions, and with the cathode and anode electrode solution being an aqueous sodium hydroxide solution.

Advantageously, the initial conductivity of the electrode solution - sodium hydroxide - is 20 mS/cm.

Advantageously, the sodium lactate solution in the feed has a concentration of 50 - 200 g/L, more advantageously 50-130 g/L and most advantageously 100 g/L.

Advantageously, the process is carried out at a limiting current density in the range of 50-500 A/m².

The lactic acid solution obtained by the method is advantageously obtained by 9.9-99.9% lactate conversion.

The current efficiency of a unit electrodialysis process is preferably in the range of 9.9-99.9%.

Also an object of the invention is a method of obtaining lactic acid from fermentation broth, using sucrose as the sole carbon source, comprising the following steps:
a. conducting bacterial fermentation with pH control in the culture medium,
b. centrifugation to separate the solid residues of the fermentation from the sodium lactate solution,
c. microfiltration in a membrane system,
d. membrane nanofiltration,
e. adsorption on activated carbon,
f. electrodialysis carried out as defined above,
g. concentration.

Advantageously, the fermentation process a. is carried out on a medium containing molasses as the sole sugar source, the pH of the fermentation process being regulated at 5.0 - 7.5, advantageously 6.5, by dosing a sodium hydroxide solution, which is derived from the concentration of the product of the electrodialysis process.

Advantageously, the fermentation process a. is carried out in a medium containing corn steep liquor as a supplementary additive.

The fermentation process a. is preferably carried out in a medium containing sugar at a concentration of 1-200g/L with a supplementation in terms of the molasses content of the medium, i.e.: corn steep liquor0.5-75.0 g/L, magnesium sulphate 0.05-2 g/L, manganese sulphate 0.005-0.5 g/L.

The nanofiltration process d. results in an initial colour reduction to <400 APHA.

The solution after the adsorption step on activated carbon e. favourably level <100 APHA. Advantageously a level of <50 APHA and more advantageously≤ 15 APHA.

Preferably, the solution after the adsorption step on activated carbon e. has a colour of 10 APHA.

Advantageously, the method of obtaining lactic acid is characterised by the fact that between the adsorption step on activated carbon e. and the electrodialysis step f. an optional purification step on ion exchange beds is carried out.

After the electrodialysis step f. the concentration g. of the obtained lactic acid solution is preferably carried out to a concentration of 15 to 99%, preferably ≥76%.

The object of the invention is also a method of obtaining lactic acid comprising the following steps:
a. membrane nanofiltration,
b. adsorption on activated carbon,
c. electrodialysis carried out as defined above,
d. concentration.

Advantageously, after the electrodialysis step c. the concentration d. of the obtained lactic acid solution is carried out to a concentration of 15 to 99%, preferably ≥76%.

Advantageously, the method of obtaining lactic acid is characterised by the fact that between the adsorption step on activated carbon b. and the electrodialysis step c. a purification step on ion exchange beds is carried out.

Advantageously the solution after the adsorption step on activated carbon b. has a colour of <100 APHA. Advantageously <50 APHA and more advantageously≤ 15 APHA. Preferably, the solution after the adsorption step on activated carbon b. has a colour of 9 APHA.

The invention presents, among other things, a method for the production and separation of lactic acid from actual fermentation broth, which contains molasses as the only carbon source. In addition to the typical fermentation, separation and concentration processes used, the authors of the invention have developed and applied an adsorption process on activated carbon for the removal of colour compounds in synergy with a three-chamber bipolar electrodialysis technique ensuring efficient conversion of lactate salt to lactic acid. As a result of the unit processes carried out according to the developed technology, concentrated lactic acid of high chemical and optical purity is obtained. The result of the invention is an elaborated solution, which provides a product of high purity, while maintaining the profitability of the industrial process and the absence of a negative impact on the environment, as well as the desirable absence of a multi-stage procedure for separation and purification of the product. The lactic acid obtained with this technology can be successfully used as a precursor for polymerisation.

### GENERAL DESCRIPTION OF THE FIGURES

The invention is illustrated in the drawings where:
Fig. 1 shows a general block diagram of an example of how lactic acid can be obtained according to the first variant.
Fig. 2 shows a detailed diagram of the technology of obtaining lactic acid according to the inventive method in the first variant.
Fig. 3 shows a detailed diagram of the technology of obtaining lactic acid according to the inventive method in variant two.
Fig. 4 shows a detailed diagram of the technology of obtaining lactic acid according to the inventive method in variant three.
Fig. 5 shows a diagram of the membrane arrangement in the electrodialysis device according to the invention.
Fig. 6 shows a comparison of energy consumption during the three-chamber bipolar electrodialysis process, depending on the process conditions used.
Fig. 7 shows the dependence of lactate flux conductance in the feed, stack voltage and lactic acid concentration during three-chamber bipolar electrodialysis using a feed with an initial lactate concentration of 100 g/L.

### DETAILED DESCRIPTION OF THE INVENTION

The method according to the invention can be implemented in at least several variants as described below. However, the scope of protection is not limited to these variants. Within the scope of the invention is any variant of the technology understood as a set of unit processes, where an electrodialysis process occurs under the conditions discussed in detail herein.

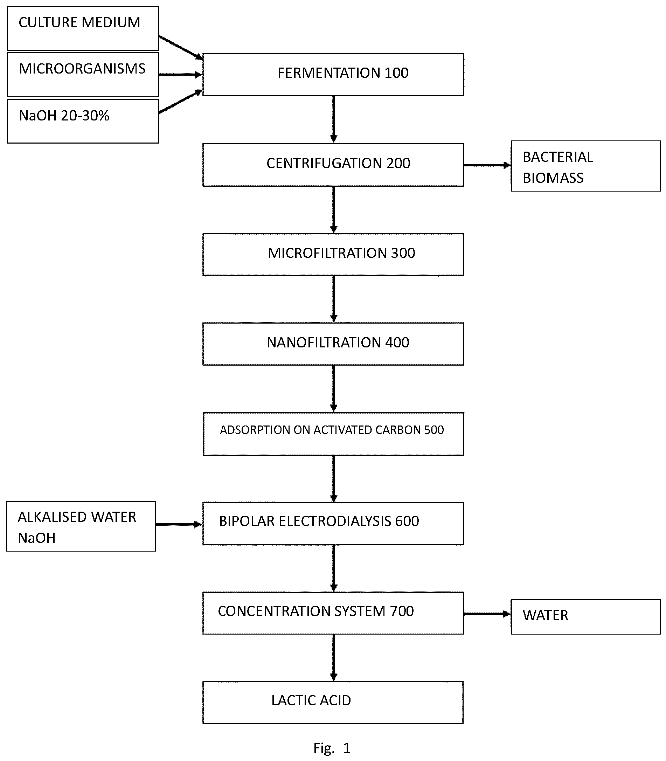

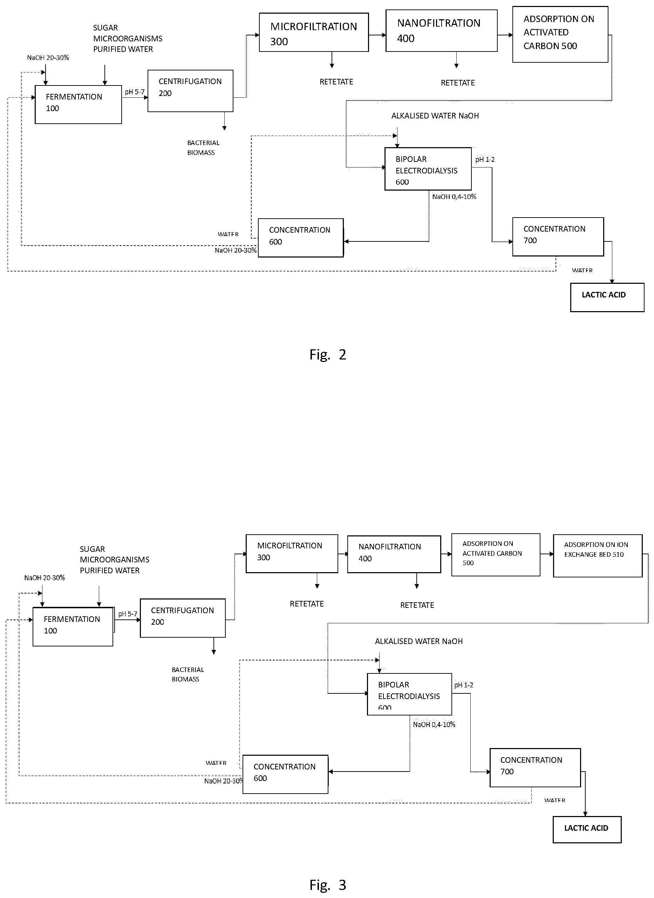

The basic variant (variant one), shown in block form in Fig. 1 and in detail in Fig. 2, comprises the following unit processes: **fermentation, centrifugation, microfiltration, nanofiltration, adsorption on activated carbon, bipolar electrodialysis, concentration.** In most cases, more concentrated solutions are advantageous for subsequent use or storage, hence the inclusion of a concentration step in the technology in question.

The second variant of the invention is the technology of obtaining lactic acid shown in Fig. 3, comprising the following unit processes: **fermentation, centrifugation, microfiltration, nanofiltration, adsorption on activated carbon, adsorption on ion exchange bed, bipolar electrodialysis, concentration.** And in this case, concentration applies to the remarks as above.

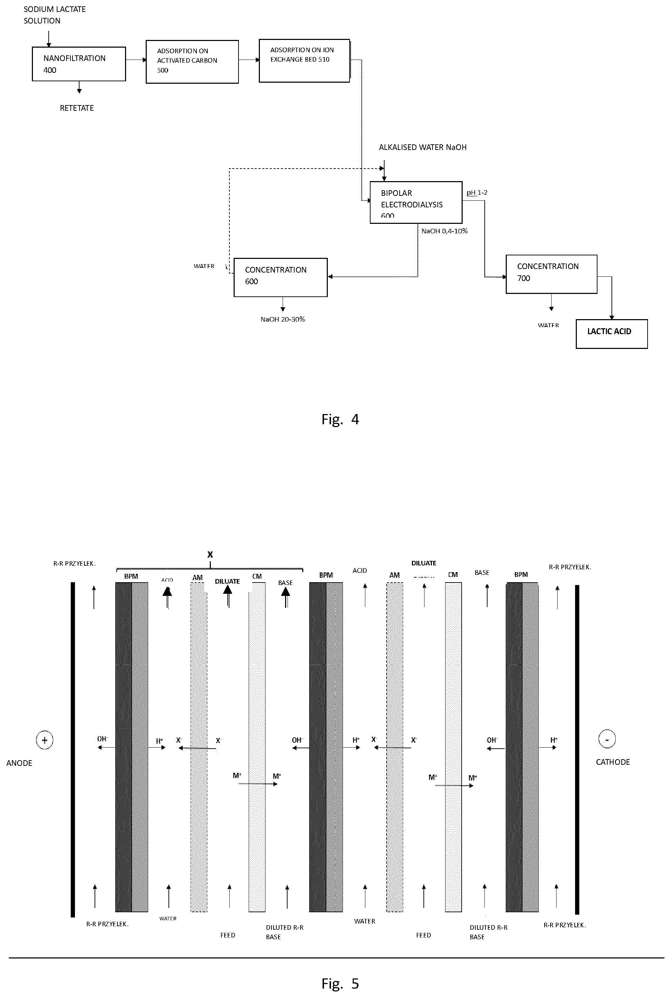

The third variant of the invention is the technology of obtaining lactic acid shown in Fig. 4 involving the following unit processes: **nanofiltration, adsorption on activated carbon, adsorption on ion exchange bed, electrodialysis, concentration.** This is a variant of the invention which involves the use of commercial lactate salt solutions.

A fourth variant of the invention is the technology of obtaining lactic acid, the course of which is also reflected in Fig. 1, comprising the following unit processes: **fermentation, centrifugation, microfiltration, nanofiltration, adsorption on activated carbon, electrodialysis, concentration,** but where the fermented input solution is carbohydrate raw materials other than molasses, which are a source of sucrose, the feedstock entering the process can be processed in an analogous method to lactic acid.

The first step in the method of obtaining lactic acid according to the invention (apart from variant 3) is the fermentation process.

Fermentation is the process by which sugar contained in a culture medium is converted to lactic acid by means of bacteria. The microorganisms used in the present invention belong to the LAB *(Lactic Acid Battery),* i.e. lactic acid bacteria, which in the described technology produce a product by homofermentation.

It is well known that microorganisms in this group can utilise a variety of carbohydrate sources. Glucose, fructose, starch hydrolysates [3] and lignocellulosic hydrolysates [4], less commonly lactose [5] and sucrose [1] can be used in the fermentation process. Sources of these sugars include agricultural waste, by-products from the food industry or residues from the dairy industry. In addition to the carbon source, lactic acid bacteria need supplements in the medium for optimal growth in the form of an additional source of nitrogen and vitamins, especially B vitamins, and salts that provide ions necessary for proper metabolic processes. Standard MRS culture medium [6], (e.g. MRS Broth Sigma-Aldrich, MRS Oxoid), used for selective culture of lactic acid bacteria typically contains glucose, which is easily assimilated by the bacteria, as a carbon source, as well as growth-promoting supplements. The correct composition of the culture medium allows the cost of raw materials to be optimised and ensures that the propagation and fermentation process is carried out efficiently. In order to reduce the cost intensity of the process, the inventors of the invention replaced the components of the standard MRS medium with another source of carbon and auxiliary substances. In the technology in question, the exclusive source of sugar in the culture medium is sucrose, supplied to the medium together with beet molasses. This by-product from the sugar industry contains up to 50% sucrose, which is metabolised by microorganisms to lactic acid less frequently than glucose. Molasses is also an additional source of B vitamins in the prepared culture medium, which has a beneficial effect on the condition of the microorganisms. It should be mentioned, however, that in addition to the beneficial components, this raw material also contains compounds that can negatively affect the purification process of the manufactured product. For this reason, molasses is not commonly used in lactic acid production. The limitations are caused by the colouring substances contained in molasses, organic acids, especially pyroglutamic acid. It was this molasses-based culture medium that the inventors decided to use in their invention. In addition to molasses, the culture medium contains additional supplements to support microbial growth, such as corn steep liquor and essential magnesium and manganese salts. The use of these ingredients in sufficient quantity allowed the inventors to completely eliminate typical supplements, such as peptone, which significantly increase the cost of the medium.

The microbial fermentation process requires sterile conditions and strict control of process parameters such as temperature, pH, pressure. As the process progresses, the pH of the culture medium changes as a result of the metabolic transformations taking place. Too low a pH, which results in an increasing concentration of lactic acid, has an inhibitory effect on the metabolism of the microorganisms and slows down further acid production. In order to maintain high productivity, it is common practice to keep the pH constant by adjusting it. In order to obtain the most efficient product, a metal hydroxide of group I of the periodic table of elements is added to the broth during the process. In the case of the invention, this is sodium hydroxide.

The sodium hydroxide dosed to maintain the appropriate pH during fermentation comes partly from the electrodialysis process (5.0 - 100%, preferably in >30% of the volume used). The concentration of sodium hydroxide obtained from the electrodialysis process is between 0.1 and 30.0% (preferably 0.4 - 10.0%, most preferably 0.4 - 4.0%) so such a solution has to be concentrated to a concentration of between 4.0 - 100.0%, preferably to a concentration of 20.0 - 30.0%. The pH value the fermentation process is maintained at 5.0 - 7.5, preferably 5.5 - 7.0, most preferably 6.5 by dosing the above NaOH solution with an automatic dosing and measuring system.

The process according to the invention is carried out in a manner typical of lactic fermentation, for example according to the method described by Guyot et al. in 2000 [7] or Ding and Tan in 2006 [8], regulating the pH in a manner described, for example, by Hetényi et al. in 2011 [9].

Fermentation is carried out in adapted equipment. These are bioreactors. Typically, the process is carried out in cylindrical tanks made of acid-resistant steel in the biotechnology regime. Such facilities are equipped with sterilisation, sanitisation and washing functions. They are equipped with control and measurement systems to monitor process parameters (temperature, pH, pressure, presence of gases, mixing speed, presence of foam). The performance of equipment allows the process to be carried out under strictly controlled, sterile anaerobic conditions. According to the invention, the process is carried out in a typical manner on a liquid substrate, using the plunge method in a vertical industrial bioreactor with the possibility of running the process in batch or semi-periodic mode. Otherwise, in a typical manner, the lactic fermentation process can also be carried out continuously in filled fermenters or in membrane bioreactors with integrated membrane modules (dialysis, ultrafiltration, microfiltration).

The post-fermentation broth with a pH value of 5.0 - 7.5, preferably 5.5 - 7.0, most preferably 6.5, and a temperature of 36±2°C, containing 50 to 200 g/L of sodium lactate (preferably 90 to 150 g/L, more preferably 120-140g/L) and 1 to 8% (preferably 2 to 4%, typically 2.5%) of bacterial biomass including the particulate fraction, should be clarified and purified. At this step, additional undesirable characteristics are a dark brown colour of the broth and a noticeable intense odour characteristic of the raw materials used.

Biomass can be removed from the fermentation broth by centrifugation and/or filtration. Flocculation methods are also known to be used to separate biomass from lactic acid-containing fermentation products (WO2004038032A1).

In the technology in question, centrifugation is used as the first step to remove biomass, leading to an effective reduction in microbial biomass and raw material solids. The use of the centrifugation step allows for better efficiency and effectiveness of subsequent steps, which are membrane filtration processes. The process results in two streams: the biomass and the supernatant for further purification. The by-product stream from the centrifugation process contains microbial biomass and can be used as an animal feed additive or in agriculture as a fertiliser, following microbial inactivation. At the same time, there is no significant loss of sodium lactate at this step. The separation process is carried out using an industrial centrifuge. A disc separator was used in the process, however, flow and decanter centrifuges are also successfully used in this type of process (examples: CEPA Z101 from Carl Padberg, Separator from Flottweg).

The centrifugation is carried out in a typical manner known from the state of the art, for example as described by Chou and Weimer in 1999 [10]. The centrifugation process according to the invention uses cyclic shunting of the biomass, without temperature control of the flowing medium.

The resulting supernatant contains 45 - 195 g/L of sodium lactate (preferably 85 - 145 g/L), has an intense odour, a suspended solids content of less than 0.5 % and a dark brown colour, its pH is 5.0 - 7.0 and its temperature is 40±2°C.

The temperature of the post-fermentation medium during the centrifugation process increases due to the heat release caused by the operation of the machine. This is beneficial for the optimum temperature of the next step, the membrane filtration process. Thanks to the sequence of operations used and the lack of temperature control during the centrifugation process, the process is less energy-intensive

The pre-clarified stream then undergoes a microfiltration process.

Microfiltration is another purification step in the method according to the invention. It is carried out in a membrane system. This process uses a ceramic membrane system with a pore size of 0.2 µm, manufactured from the material α- Alumina. Ceramic membranes are easier to disinfect compared to alternatively used polymeric membranes [11]. The use of a combination of centrifugation and microfiltration processes effectively provides an efficient means of separation. In this method, all suspended residual contaminants, i.e. residual bacterial biomass and particulate matter, are eliminated. The classical microfiltration process carried out in conventional filters in direct flow filtration mode also ensures that the next clarification step of the supernatant e.g. CRM3019-200nm (BTS Engineering) takes place correctly. The method according to the invention employs cross-flow filtration, in a continuous process in which the feed stream flows parallel to the membrane filtration surface and generates two outgoing streams. The developers used an innovative cross-flow solution in which the filter channels of the ceramic membrane have a special star shape (Mantec Filtration). This shape has a positive effect on the filtration process by increasing the filtration surface area and the turbulence caused at lower tangential flow velocities. The implemented solution results in a reduction in pumping energy requirements, therefore improving the cost-effectiveness of the process.

The resulting permeate contains 45 - 195 g/L sodium lactate (preferably 80-140 g/L), has a pH of 5.0 - 7.0 and a temperature of 40±2°C. The permeate is clear, but still has an intense odour and a dark brown colour.

By means of nanofiltration, all impurities larger than sodium lactate, especially residual sugars, are removed. The system contains a spiral membrane system *(cut-off* 200-300 Da). Spiral membranes such as, for example, TRISEP^{®} PLT 4040-TS40-31 from Lenntech or NF90-4040, NF90-2540 from Filmtec Membranes or RO90-8038/30 from Alfa Laval will be used in the process.

The nanofiltration process removes multivalent metal ions, excessive amounts of which are critical for the next process step - their content in the permeate after nanofiltration must not exceed 10 ppm. Thus, a >90% reduction in the content of divalent and trivalent cations (Mg²⁺, Ca²⁺, Fe³⁺) is achieved with respect to the initial solution after microfiltration. The selectivity of the membranes in this process **ensures low retention of lactate ions,** which is often problematic in industrial processes. Another desirable effect of the nanofiltration process is the **initial colour reduction of the feed.** By comparing the main stream of the real solution before the nanofiltration process and the stream after the process, we are talking about obtaining from the dark brown real solution after the microfiltration process (>500 APHA), a product with a delicate straw yellow colour (in the range of 150-400 APHA, preferably 150-350 APHA, most preferably 150-250 APHA). Since the parameters of the nanofiltration solution were beyond the APHA scale, the colour changes were also analysed by measuring the absorbance of the stream before and after the process (spectrophotometric measurement at 420nm). From the results obtained, a significant colour reduction of at least 98.5 %(for the post-fermentation broth) was observed.

The permeate obtained contains 40 - 185 g/L of sodium lactate (preferably 75 - 135 g/L), its pH is 5.0 - 7.0 and its temperature is 40±2°C. The resulting permeate is clear, straw yellow in colour and has a slightly noticeable characteristic odour.

The clarified solution with the pre-reduced colour intensity is subjected to a decolourisation step, preferably by treatment with activated carbon.

The main task of the carbon bed is to sorb the remaining colour compounds from the lactate solution and thus reduce the colouration of the permeate after the nanofiltration process to <100 APHA (preferably <50 APHA, even more preferably ≤ 15 APHA). The activated carbon that can be used according to the invention is a particulate activated carbon (PWA) - NORIT SX2 analitical purity additionally other commercial carbons can be used, e.g. Norit Darco 12x40. Surprisingly, the creators of the invention achieved a high reduction in colouration while not having to direct the same stream repeatedly onto the carbon column (multiplying the contact of the medium with the carbon bed). This operation also has a positive effect on reducing the undesirable but occurring retention of lactate ions by the carbon bed at this step. Optimally applied activated carbon has a developed mesoporous structure, a specific surface area of 1050 m2/g (BET) and a particle size of 12x40 mesh (1.70 - 0.40 mm). The achieved effect has a positive impact on the economic and environmental aspects of the process. For 1 tonne of molasses processed, 220 kg of activated carbon is needed for the reduction of colour compounds (melanoidin), preferably by at least 87%. The service life of the carbon granulate used has also been determined and can be successfully regenerated. The feed flow rate through the carbon bed is 100 - 1000 L/h, preferably 300-600 L/h.

On the basis of the processes and experiments carried out, the effectiveness of the colour reduction of the medium using the carbon bed column was determined. An additional result observed organoleptically is the retention on the activated carbon bed of odour compounds coming from the main source of sugars, thus completely eliminating the characteristic molasses odour in the final product.

Using the APHA scale and referring to the actual solution obtained after fermentation, whose initial colour exceeds 500 APHA, after decolourisation on a carbon bed, 5 to 50 APHA (preferably 5 - 15 APHA) is obtained, which represents at least 90% colour reduction (taking as a starting point the colour >500 APHA occurring after fermentation). Advantageously, the decolourisation occurs at a level of 97 - 99%. Furthermore, the obtained stream contains 37 - 175 g/L of sodium lactate, preferably 70 - 130 g/L, more preferably 80 - 130 g/L and most preferably 100 g/L, its pH is 5.0 - 7.0, its odour is not perceptible.

The real post-fermentation solution prepared in this method is suitable for processing using current processes.

If the solution after adsorption of the coloured compounds onto the activated carbon does not meet the purity requirements of the electrodialyser feed (which may be due to the quality of the raw materials used in the fermentation), in particular if the concentrations of multivalent metal ions (Ca, Mg, Fe, Zn, etc.)) exceed the critical limits permissible for the electrodialysis stack described by the characteristics of the membranes used, or the solution contains other organic acids, then it is necessary to reduce the content of such chemical entities to acceptable levels on the ion exchange columns (as in variants 2 and 3 detailed above). The execution of such a process is carried out in a manner typical and familiar to an expert in the field. The bed can be a chelating resin, e.g. Purolite S940, or a cascade of cationite and anionite columns, e.g. from Lewatit. The feed flow rate through the ion exchange column system is 100-1000 L/h, preferably 300-600 L/h, preferably 400 L/h.

The electrodialysis process is carried out according to the invention in an electrodialysis with a three-chamber bipolar electromembrane module (EDBM). As a result of electrodialysis, three streams are produced, two concentrates in the form of products - an impurity-free lactic acid solution and a second product, i.e. sodium hydroxide solution, and a depleted diluate solution in the form of a desalted feed stream. The three-chamber system ensures complete separation of the product and separation from the rest of the impurities present in trace amounts in the feed solution (e.g. other organic acid anions, sugar residues or metal ions). The stacking arrangement prevents the alkaline solution from coming into direct contact with the acidic solution, thus preventing unwanted uncontrolled migration of cations.

A schematic of the electrodialysis process is shown in Fig. 5. The feed stream containing purified aqueous sodium lactate solution is labelled FEED in the schematic, is transformed as a result of the process and is received as ion-depleted DILUATE.

The alkali stream constituting the electrode solution is denoted as R-R PRZYELEK. During the process, from the stream of demineralised water alkalised with a small amount of sodium hydroxide(to a conductivity of 20 mS/cm), designated as DILUTED R-R BASE, a more concentrated stream of sodium hydroxide, designated as BASE, is formed, which is collected as a product, while part of the stream is returned to the R-R PRZYELEK. stream and recirculates in the system as described later in the description.

The demineralised water stream, into which lactate ions migrate during the process and lactic acid is formed, was designated ACID.

BPM stands for bipolar membrane, CM for cation exchange membrane and AM for anion exchange membrane.

The resulting sodium hydroxide solution is reused in the manner as described below.

Recycling of the sodium hydroxide stream for the fermentation process is possible after its prior concentration. For this purpose, an alkali stream with a concentration of 0.1 to 30.0%, preferably 0.4 - 10.0%, most preferably 0.4 - 4.0%, obtained after the electrodialysis process is directed to an evaporator and concentrated in a typical manner until a concentration of 20 to 30% is reached. The water evaporated during the concentration process can be reused in the electrodialysis process.

The sodium hydroxide stream recycle is also used as an electrode solution in the electrodialysis process.

The sodium hydroxide stream recycle can also be used for washing plants.

The use of a bipolar membrane electrodialysis process in a three-chamber membrane stack system, enabling the processing of a feed stream rich in lactic acid salt (37-175 g/L sodium lactate, preferably 70-130 g/L and most preferably 80 - 130 g/L), obtained by fermentation and efficient broth clarification, makes it possible to separate organic and inorganic salts and produce separated acid and base streams of high quality.

In addition, the production of fermentation broth with a high lactate salt content after the purification steps and the use of the EDBM process allows the elimination of the concentration step using monopolar electrodialysis, which is very typical in state-of-the-art technologies. According to many literature studies [12-15], electrodialysis with a monopolar module is a commonly used component of the lactic acid production process pathway for the concentration of lactate in the stream directed to the conversion process. This step generates significant amounts of waste, such as sludge and mineral acids, which must later be managed or disposed of.

The bipolar membranes used in the process can consist of two integrated membranes e.g. from Veolia, one anion exchange AR103N and the other cation exchange CR61P, commercially available bipolar membranes e.g. FBM-PK-130 from Fumatech BWT GmbH, EDBM-Z/10-0.8 from MemBrain Mega S.A. can also be used in the solution, PC bip-ED from PCCell, BP-1E from Tokuyama Co., etc., if their operating parameters (size, pH range, operating temperatures, etc.) allow the construction of a compatible electrodialytic stack with other stack components. On the other hand, AR204 from Veolia, Neosepta AMX from Tokuyama Co., PC 200 D-ED from PCCell, RALEX AMHPP from MemBrain Mega S.A., FAB-PK-130 from Fumatech BWT GmbH, etc. can be used as anion exchange membrane. The cation exchange membrane can be membranes such as PC SK-ED from PCCell, CR63TP from Veolia, RALEX CMHPP from MemBrain Mega S.A., FKB-PK-130 from Fumatech BWT GmbH, etc. The individual membranes should be separated by typical spacers, for example from Veolia ED-SPACER, which is made of polypropylene; spacers of other typical materials can also be used in the process to ensure safe operation under process conditions and the correct direction of the streams. The cathode and anode can be typical electrodes made of materials such as ruthenium- and/or iridium-coated titanium, platinum containing metal oxide layers or 316 stainless steel, or Hastelloy C steel and other standard electrode materials.

In the process of bipolar electrodialysis, it is known that an electric current is used to differentiate the ionic constituents of a solution across anion-selective and cation-selective membranes and to split water molecules on bipolar membranes, resulting in the formation of hydroxyl (OH⁻) and hydroxonium (H₃O⁺ labelled in Fig. 5 as H⁺) ions. The ions are transported across bipolar membranes, (labelled BPM in Fig. 5) made up of cation- and anion-exchange layers, to adjacent acid and base chambers. In the acid chamber, the conversion of lactate ions to the acid form takes place (according to Fig. 5: X⁻ + H⁺ → ACID). Similarly, hydroxide ions migrate to the alkali chamber, causing an increase in the alkalinity of the solution, where they meet metal ions transported across cation-exchange membranes to form a base - NaOH (according to Fig. 5: M⁺ + OH⁻→ BASE). The X represents the basic module of the cell pair, which, when duplicated 5 to 100 times, forms a stack.

The state of the art with regard to lactic acid production involving electrodialysis processes does not always focus on optimising the current conditions occurring during electrodialysis in the context of saving the energy efficiency of the process related to energy losses.

According to the invention, the electrolyte solution, the so-called electrode solution, is a soda lye solution with a nominal conductivity of 20 mS, which recirculates in the electrode spaces and is supplemented, if necessary, with an alkali solution produced by electrodialysis. The electrode solution washes over the cathode and anode and is only found in the electrode spaces. The concentration of the electrode solution ranges from 0.4% to 8.0%, preferably 0.4-4.0%.

It has been experimentally established, as explained in detail in the examples, that carrying out three-chamber bipolar electrodialysis to convert and separate lactic acid from a sodium lactate solution using sodium hydroxide as the electrode solution at concentrations of 0.4% to 8.0%, preferably 0.4-4.0%, for both the cathode and anode, leads to the surprising effect of reducing the energy intensity and increasing the efficiency of the process.

The vacuum concentration process was carried out to concentrate the lactic acid solution obtained after the EDBM process. Vacuum concentration processes were carried out using a vacuum evaporator in a typical manner to a concentration of 15 to 99%, preferably ≥ 76%. Concentration can be realised by classical reduced pressure distillation techniques (US6489508), short path *evaporation* [16-19] or other well-known techniques.

After the process sequence described above (according to all variants), a lactic acid solution with a purity of 70 - 99%, preferably > 90%, even more preferably > 95% and a concentration of 15 - 99%, preferably ≥76%, is obtained.

A schematic diagram of the installation according to the various variants of the invention is shown in Fig. 2, 3 and 4. Fig. 1 in turn presents a block diagram of variant one. Culture medium, microorganisms and NaOH 20-30% are introduced into the fermentation bioreactor (100). The fermentation broth is then transported to the centrifugation process (200), where the biomass is separated. The biomass is a by-product that can be further managed. The supernatant, in turn, is transferred to a further process, microfiltration (300), where residual biomass and solids are separated. The permeate is transported to a nanofiltration process (400) and then to an activated carbon adsorption process (500) for further purification. The decolourised sodium lactate solution with highly reduced ion content, as a feed, is directed to the electrodialysis process (600), where a NaOH solution is introduced and then to the concentration (700), from where a pure concentrated lactic acid solution is received. A by-product of the concentration process is water, which can be managed as part of the process.

Fig. 2 shows a block diagram of the plant according to the first variant of the invention. In this variant, the culture medium with microorganisms and dosed NaOH 20-30% is introduced into the fermentation bioreactor (100). The fermentation broth is then transported to the centrifugation process (200), where the biomass is separated. The biomass is a by-product that can be further managed. The supernatant, in turn, is transferred to a further process, microfiltration (300), where residual biomass and solids are separated. The by-product is a filtered retentate that can be further processed. The permeate is transported to the nanofiltration process (400) for further purification. The by-product is filtered retentate available for further management. The main stream is then directed to an adsorption process on activated carbon (500) for colour reduction. The decolourised sodium lactate solution with highly reduced ion content as feed is directed to the electrodialysis process (600), where a NaOH-alkalised water solution is introduced. The 1-4% NaOH solution obtained in this process is reused within the technology, having been concentrated (610). It is used to regulate the pH in the fermentation process (100). The water stream obtained from the concentration operation (610) is reused in the bipolar electrodialysis process (600) to prepare NaOH-alkalised water. The main stream is then diverted to the concentration (700), from where a pure concentrated lactic acid solution is received. The by-product stream of the concentration process is water, which is disposed of as part of the fermentation process (100).

The order or number of process steps in Fig.3 and Fig.4 varies accordingly with respect to what is described in the detailed description of the technology in question and its variants. In Fig.3 according to the second variant of the invention, between the step of adsorption on activated carbon (500) and bipolar electrodialysis (600), adsorption on an ion exchange bed (510) is used. In Fig.4, the first element according to of the third variant of the invention is nanofiltration (400), followed sequentially by adsorption on activated carbon (500), adsorption on ion exchange bed (510), bipolar electrodialysis (600) and concentration (700) of lactic acid. The water stream obtained from the concentration operation (610) is reused in the bipolar electrodialysis process (600) to prepare NaOH-alkalised water, and the 20-30% NaOH-concentrated stream obtained from the concentration operation (610) is an additional product.

### Abbreviations used:

LA *- lactic acid*
PLA *- polylactic acid*
MRS *- deMan-Rogosa-Sharpe*
EDBM *- bipolar electrodialysis*

Unless otherwise mentioned, the temperatures given in the description are to be understood as +/- 2° C.

Unless otherwise stated, the flow rates given in the description are to be understood as +/- 5 L/h.

Unless otherwise stated, the processes given in the description were carried out at atmospheric pressure.

Unless otherwise stated, the pressure values given in the description in units are to be understood as +/-0.1.

**The following technical and performance characteristics were obtained through the processes used:**
∘ An economically advantageous method of obtaining lactic acid, ensured by the energy efficiency brought about by reducing the operations used and the judicious use of the thermal energy of the streams in the designed process line. The typical carbon source of the culture medium (glucose) was replaced by a carbon source derived from molasses (sucrose), which is a by-product of the food industry and problematic for use in this type of process. Concerns about the hard-to-remove colouring and odour of the product have been eliminated in the claimed process. The inventors of the invention have developed an efficient method of obtaining a pure product by converting the sugar supplied to the substrate solely from molasses - a reluctant carbon source. Costly components of a typical culture medium were eliminated by using alternative raw materials. The substrate composition used provides the basis for efficient lactic fermentation.
∘ The individual unit processes in the process line are designed and optimised to achieve very good, or at least satisfactory, mixture processing parameters by running the individual operations at the operating temperatures of the respective stream. The recuperation of heat between the streams allows significant energy savings and removes the need for additional heating of the mixture prior to steps requiring operation at elevated operating temperatures. In addition, the refined operating parameters of selected processes (e.g. centrifuge and electrodialysis) allow simultaneous heating of the working streams as a side-effect of running the process at set equipment operating settings, so that the following continuously running step does not require an additional heating element.
∘ The use of an uncomplicated and only 4-step purification process: W - MF - NF - WA - EDBM results in high purity lactic acid from the actual solution, of which molasses is a component and the base raw material. The technological steps used produce a pure, clear product with a highly reduced colour (<50 APHA), without the characteristic odour from the raw materials used.
∘ The combination of an efficient fermentation, which yields a fermentation broth of advantageously 90 to 150 g/L, more advantageously 120-140g/L sodium lactate, and appropriately optimised purification steps with high yield and efficiency, makes it possible to direct the actual solution directly to the bipolar electrodialysis step, without the need for additional broth concentration. This is in contrast to other technologies, which typically use an additional step of concentrating the lactate salt solution before the actual step of converting the dissociated form of lactate to acid.
∘ The efficient adsorption process on the carbon bed allows the preparation of a feed of optimum composition for the electrodialysis process, with low lactate retention.
∘ Implementation of an alkali solution, which is also a product of electrodialysis, as an electrode solution in a bipolar electrodialyzer. The modification improves the electrodialysis step, eliminates the need for an additional chemical substance as an electrode solution medium, furthermore provides a form of electrodialysis product management by recirculating part of the product stream in the circuit, and additionally enables high process efficiency to be maintained and has a beneficial effect on energy consumption parameters during the process.
∘ Improved electrodialysis efficiency as a result of replacing traditional electrode solutions (H₂SO₄, Na₂SO₄ solution) with sodium hydroxide solution. The direct use of the process stream obtained in electrodialysis as an electrode solution surprisingly increases the degree of conversion of lactate to lactic acid and is characterised by higher current densities at which the electrodialysis process can be carried out; in addition, the unit time efficiency of lactic acid yield is increased, which also translates into lower electricity consumption in the time required to produce 1 kg of lactic acid.

### EXAMPLES

**The method according to the invention is illustrated by the following examples.**

### Example 1. Process line according to the first variant

The solution of the actual fermentation broth was obtained by fermentation of molasses by microorganisms of the genus *Lactobacillus,* carried out in a typical state-of-the-art manner, at 37°C maintaining pH values of 6.5.

The process used a culture medium with the following composition: sugar at a concentration of 140 g/L, corn steep liquor 10 g/L, magnesium sulphate 0.2 g/L, manganese sulphate 0.05 g/L. The fermentation broth, 500 L in volume, with a pH value of 6.5 and a temperature of 37°C, containing 135 g/L of sodium lactate, was subjected to separation of microbial biomass by centrifugation carried out with a disc centrifuge at a feed flow rate of 0.80m³/h, with a rotational frequency of 7000 rpm, using cyclic shunting of the biomass, without temperature control of the flowing medium. The resulting supernatant, with a pH of 6.5 and a temperature of 40°C, was subjected to microfiltration and nanofiltration, which were carried out in a typical state-of-the-art manner. The microfiltration process was carried out in a system equipped with a ceramic membrane with a pore size of 0.2 µm (Mantec Filtration), maintaining a transmembrane pressure of 4-6 bar during the process, with an average of 5 bar. The permeate was nanofiltrated on a 300 Da *cut-off* polymer spiral membrane system (TAMI), maintaining a transmembrane pressure in the range 20-26 bar during the process, averaging 23.66 bar. Subsequently, decolourisation was carried out on activated carbon according to the method outlined below in Example 2. Bipolar electrodialysis was carried out as detailed in Example 5. A 100 g/L lactic acid solution was concentrated on evaporators using a temperature of 55° C and a pressure of 25 mbar.

### Example 2: Activated carbon adsorption step

After an initial colour reduction by nanofiltration, the resulting permeate is directed to a carbon column for further reduction of colour compounds. A stream with a colour of 270 APHA, lactate content of 111 g/L, pH 6.0 was fed onto the column at a flow rate of 400 L/h. The activated carbon used has a developed mesoporous structure, a specific surface area of 1050 m²/g (BET) and a particle size of 12x40 mesh (1.70 - 0.40 mm). In the example described, 1 unit volume of activated carbon was used per 20 units of volume passed through the feed column. After the process, a stream was obtained, characterised by a colour of 10 APHA, a content of 100 g/L sodium lactate, a pH of 5.5 and no perceptible odour. For 1 tonne of molasses processed, 220 kg of activated carbon was used to reduce the colouration without the need for activated carbon regeneration. This is achievable due to the optimised flow rate and the absence of the need to pass the solution through the activated carbon column multiple times.

### Example 3. Application step of ion exchange resins

In order to ensure the appropriate parameters, i.e. the concentration of multivalent metal ions and the content of other organic acids, the feed containing sodium lactate obtained by bioconversion, after a purification process by centrifugation, microfiltration, nanofiltration, adsorption on activated carbon techniques, realised according to examples 1 and 2, can be directed to ion-exchange bed columns. Each of the four columns was filled with 25 kg of bed. The feed flow rate through the ion exchange column system is 400 L/h. First, the feed is passed through the cation exchange bed using a strongly acidic resin based on styrene-divinylbenzene copolymer (Purolite). The product after the cation exchange bed column is then directed to a column with a weakly basic anion exchange resin bed based on styrene-divinylbenzene copolymer (Levatite). After passing through both beds, the feed directed to the electrodialysis step contains less than 10 ppm of multivalent ions and the process is carried out according to Example 5. The final step of obtaining lactic acid is then concentration on evaporators as described in Example 1.

Example 4. Application of the invention to obtain lactic acid from commercial sodium lactate The method according to the invention then includes the steps: nanofiltration, adsorption on activated carbon, adsorption on ion exchange bed, EDBM, concentration.

A model aqueous solution of sodium lactate of known composition was also processed according to the described technology. A commercial product of at least technical quality with a sodium lactate content of 115 g/L was subjected to a nanofiltration process according to the method described in example 1. Subsequently, decolourisation on activated carbon was carried out according to the method described in example 2, with the difference that 36 units of volume passed through the feed column were used per 1 unit volume of activated carbon to achieve the same effect. After the process, a stream was obtained, characterised by a colour of 9 APHA, a content of 104 g/L of sodium lactate and a pH of 5.5. The step of using ion exchange resins did not differ from the method described according to example 3. Bipolar electrodialysis was then carried out as described in example 5. A lactic acid solution of 100 g/L was concentrated on evaporators using a temperature of 55°C and a pressure of 25 mbar.

### Example 5. The electrodialysis of a feed with an initial lactate concentration of 100 g/L

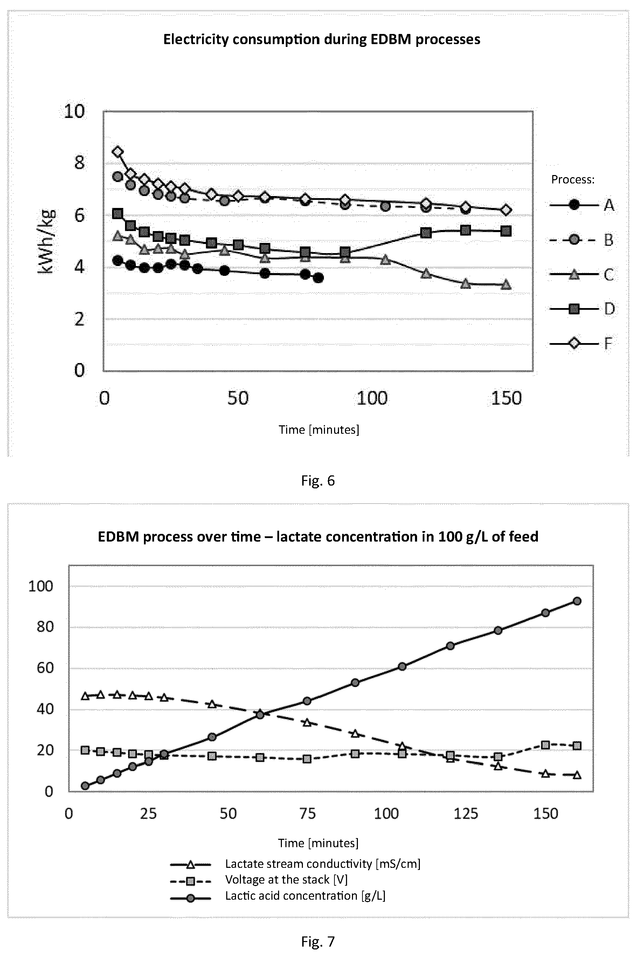

A three-chamber bipolar electrodialysis was carried out of a purified real lactate solution obtained from fermentation (carried out according to the method described in Example 1), containing 100 g/L lactate with a pH of about 5.5. The real fermentation broth obtained after purification by centrifugation, microfiltration, nanofiltration, adsorption on activated carbon and 10 APHA staining techniques was subjected to bipolar membrane electrodialysis. The actual and purified fermentation broth was introduced into the feed/diluate chamber, a receiving solution based on demineralised water was introduced into the acid chamber, and a NaOH solution with a conductivity of 20 mS/cm prepared on the basis of demineralised water was introduced into the alkali chamber, which also acts as an electrode solution. The stack used in the example, as shown in Fig. 5, was equipped with alternating bipolar membranes (BPM), anion exchange membranes (AM) and cation exchange membranes (CM) in the number of 10 cell pairs (X) with an active area of 0.2 m², which were separated by polypropylene spacers of the electrode solution chamber (R-R PRZYELEK.), feed/ diluate (DILUATE), base (BASE) and acid (ACID). The unit is powered by a DC power source. The process was run for 160 min, at a current density of 453 A/m², with constant flow rates of the working solutions for the feed stream of 135 L/h and for the other streams of 180 L/h. The operating temperature for the membrane stack was up to 45°C. The resulting changes in transmitter flux conductivity, stack voltage and lactic acid concentration during the three-chamber bipolar electrodialysis process using a transmitter with an initial lactate concentration of 100 g/L are presented in the summary diagram Fig. 7.

Example 6. Comparison of the current values of the electrodialysis process carried out according to the invention, when a sodium hydroxide solution was used as the electrode solution, relative to a typical process, where a solution of sulphuric acid(VI) and sodium sulphate was used as the electrode solution.

A series of EBDM processes (processes A-F) of actual aqueous solutions obtained by bioconversion of molasses and subsequent treatment according to the method described in Example 1, with adsorption on activated carbon as in Example 2. In order to demonstrate from the research side the assumed effect of the invention for the electrodialysis process as described in Example 5, the following findings were carried out.

An electrodialysis process system containing an aqueous solution of H₂SO₄ and Na₂SO₄ (with a conductivity of 20 mS/cm) as the electrode solution is the reference system. A concentration of 80 g/I of sodium lactate in the feed was chosen as a reference (process F) to directly compare the parameters of this process to the invention - an electrodialysis process carried out under identical conditions to the reference process but using an aqueous NaOH solution (with a conductivity of 20 mS/cm) as the electrode solution (process B). In addition, selected parameters are shown for the processes according to the invention but at different sodium lactate concentration values: 50 g/l, 100 g/l, 130 g/l. The parameters in Table 1. were calculated using the basic and well-known relations in the field.

The EDBM efficiency is a parameter characterising the actual electrodialysis process in relation to the process result calculated from the product mass resulting from Faraday's law, the electrochemical equivalent of the conversion and the current during the process, taking into account the percentage efficiency of the actual process. The conversion rate reflects the ratio of the amount of lactate that converted to lactic acid in relation to the total lactate content of the solution expressed as a percentage. The EDBM efficiency describes the amount of acid produced per unit time, taking into account the efficiency of the electrodialysis process. The current efficiency expresses the ratio of the mass of product separated to the mass resulting from Faraday's law. Electricity consumption is based on the conversion of voltage and current on the stack during the electrodialysis process, duration and mass of product obtained, this parameter describes the cost-effectiveness of the industrial electrodialysis process per unit mass of lactic acid produced.

From an industrial economic point of view of the method electrodialysis is carried out, the higher the current output and the lower the energy consumption, which is also accompanied by a high conversion rate, the more the process can be considered economically advantageous.

The results of the findings are summarised in Table 1.

**Table 1.**

| **Process** | **Lactate concentration [g/l]** | **Type R-R PRZYELEK.** | **EDBM efficiency [%]** | **Conversion rate [%]** | **EDBM efficiency [g LA/h]** | **Current density [A/m²]** | **Process current efficiency [%]** | **Z, energy consumption [kWh/kg]** |
|---|---|---|---|---|---|---|---|---|
| A | 50 | aqueous solution of NaOH | 83.1 | 80.8 | 312 | 452.0 | 86.07 | 3.96 |
| B | 80 | aqueous solution of NaOH | 96.4 | 87.1 | 343 | 452.5 | 91.16 | 5.11 |
| C | 100 | aqueous solution of NaOH | 98.7 | 91.8 | 383 | 453.0 | 94.05 | 4.41 |
| D | 130 | aqueous solution of NaOH | 97.2 | 88.9 | 361 | 453.0 | 93.59 | 4.99 |
| F | 80 | aqueous solution of H₂SO₄ and Na₂SO₄ | 96.0 | 84.4 | 307 | 427.0 | 83.67 | 6.97 |

The use of a NaOH solution as an electrode solution, instead of the typical H₂SO₄ and Na₂SO₄ solution, results in **an improved electrodialysis step, improved efficiency** and, above all, a **surprisingly high practical conversion of lactate to lactic acid** (87.1% instead of 84.4%), whereas achieving a conversion of >86% in industrial-scale equipment is a technological challenge. Changing the electrode solution to a NaOH solution, **eliminates the need to use an additional chemical** as an electrode solution medium, furthermore **provides a form of electrodialysis product management by recirculating** part of the product stream in the circuit, and additionally **enables high process efficiency to be maintained** and **has a beneficial effect on energy consumption parameters** during the process. The direct use of the technological stream obtained during electrodialysis as an electrode solution surprisingly **increases the degree of conversion of** lactate to lactic acid up to 91.8% and is characterised **by higher current densities** at which the electrodialysis process can be carried out; in addition, **the unit efficiency of lactic acid yield over** time **increases,** which also translates **into more than 36% lower electricity consumption** over the time required to produce 1 kg of lactic acid.

The effect of the invention in the form of the improved parameters presented above is observed for different values of feed concentrations in the range 50-130 g/L.

### Example 7. The electriodiaysis of a feed with an initial lactate concentration of 130 g/L

Three-chamber bipolar electrodialysis of purified real sodium lactate solution obtained from beet molasses fermentation, containing 130 g/L lactate, pH approx. 5.5. Bipolar membrane electrodialysis was performed on real fermentation broth obtained after purification by centrifugation, microfiltration, nanofiltration, adsorption on activated carbon and 10 APHA staining techniques. The actual and purified fermentation broth was introduced into the feed/diluate chamber, a receiving solution based on demineralised water was introduced into the acid chamber, and a NaOH solution with a conductivity of 20 mS/cm prepared on the basis of demineralised water was introduced into the alkali chamber, which also acts as an electrode solution. The stack used in the example, Fig. 5, was equipped with alternating bipolar membranes (BPM), anion-exchange membranes (AM) and cation-exchange membranes (CM) in the number of 10 cell pairs (X) with an active area of 0.2 m², which were separated by polypropylene spacers for the electrode solution (R-R PRZYELEK), feed/diluate (DILUATE), base (BASE) and acid (ACID) chambers. The unit is powered by a DC power source. The process was run for 210 min, at a current density of 453 A/m², while keeping the flow rates of the working solutions constant for the 135 L/h feed stream and for the other streams at 180 L/h. The optimum permissible operating temperature for the membrane stack is up to 45°C. The parameters characterising the three-chamber bipolar electrodialysis process using a feed with an initial lactate concentration of 130 g/L are presented in Table 1, Process D.

### Example 8. The electriodiaysis of a feed with an initial lactate concentration of 80 g/L

Three-chamber bipolar electrodialysis of a purified real lactate solution obtained from the fermentation of beet molasses, containing 80 g/L lactate with a pH of approximately 5.5. The real fermentation broth obtained after purification by centrifugation, microfiltration, nanofiltration, adsorption on activated carbon and 10 APHA staining techniques was subjected to bipolar membrane electrodialysis. The actual and purified fermentation broth was introduced into the feed/diluate chamber, a receiving solution based on demineralised water was introduced into the acid chamber, and a NaOH solution with a conductivity of 20 mS/cm prepared on the basis of demineralised water was introduced into the alkali chamber, which also acts as an electrode solution. The stack used in the example, Fig. 5, was equipped with alternating bipolar membranes (BPM), anion-exchange membranes (AM) and cation-exchange membranes (CM) in the number of 10 cell pairs (X) with an active area of 0.2 m², which were separated by polypropylene spacers between the electrode solution (R-R FRAME.), transmitter/diluate (NADAWA/DILUAT), base (BASIC) and acid (ACID) chambers. The unit is powered by a DC power source. The process was run for 135 min, at a current density of 452.5 A/m², with constant flow rates of the working solutions for the 135 L/h feed stream and for the other streams of 180 L/h. The optimum permissible operating temperature for the membrane stack is up to 45°C. The parameters characterising the three-chamber bipolar electrodialysis process using a feed with an initial lactate concentration of 80 g/L are presented in Table 1, Process B.

### Example 9. The electriodiaysis of a feed with an initial lactate concentration of 50 g/L

Three-chamber bipolar electrodialysis of a purified real lactate solution obtained from the fermentation of beet molasses, containing 50 g/L lactate with a pH of about 5.5. The real fermentation broth obtained after purification by centrifugation, microfiltration, nanofiltration, adsorption on activated carbon and 9 APHA staining techniques was subjected to bipolar membrane electrodialysis. The actual and purified fermentation broth was introduced into the feed/diluate chamber, a receiving solution based on demineralised water was introduced into the acid chamber, and a NaOH solution with a conductivity of 20 mS/cm prepared on the basis of demineralised water was introduced into the alkali chamber, which also acts as an electrode solution. The stack used in the example, Fig. 5, was equipped with alternating bipolar membranes (BPM), anion-exchange membranes (AM) and cation-exchange membranes (CM) in the number of 10 cell pairs (X) with an active area of 0.2 m², which were separated by polypropylene spacers for the electrode solution (R-R PRZYELEK.), feed/diluate (/DILUATE), base (BASE) and acid (ACID) chambers. The unit is powered by a DC power source. The process was run for 80 min, at a current density of 452 A/m², while keeping the flow rates of the working solutions constant for the 135 L/h feed stream and for the other streams at 180 L/h. The optimum permissible operating temperature for the membrane stack is up to 45°C. The parameters characterising the three-chamber bipolar electrodialysis process using a feed with an initial lactate concentration of 50 g/L are presented in Table 1, Process A.

### Example 10. Application of the invention to obtain lactic acid by fermenting sucrose from a source other than molasses

The method according to the invention then includes the steps: fermentation, centrifugation, microfiltration, nanofiltration, adsorption on activated carbon, electrodialysis, concentration.

A solution of the actual fermentation broth was obtained by fermentation of sucrose from a source other than molasses by Lactobacillus microorganisms, carried out in a typical state-of-the-art manner, at 37°C maintaining pH values of 6.5. The fermentation process resulted in a solution containing 120 g/L sodium lactate. The process was carried out analogously to that described in Example 1, with the difference that at the step of the process of adsorption on activated carbon, according to the method presented in Example 2, 55 units of volume of feed passing through the column were applied per 1 unit of volume of activated carbon to obtain the same effect. After the process, a stream was obtained, characterised by a colour of 10 APHA, a content of 102 g/L sodium lactate and a pH of 5.5.

Improved electrodialysis process conditions were also observed for this example, analogous to that described above.

### Literature:

[1] Abedi, E., & Hashemi, S. M. B. (2020). Lactic acid production-producing microorganisms and substrate sources-state of art. Heliyon, 6(10), e04974. https://doi.org/10.1016/j.heliyon.2020.e04974
[2] Ojo, E. & de Smidt, O. (2023). Lactic Acid: A Comprehensive Review of Production to Purification. Processes, 11(3), 688. https://doi.org/10.3390/pr11030688
[3] Petrov, K., Urshev, Z. & Petrova P. (2008). I(+)-Lactic acid production from starch by a novel amylolytic Lactococcus lactis subsp. lactis B84. Food Microbiology, 25(4), 550-557. .
[4] Li, Y., Bhagwat, S. S., Cortés-Penã, Y. R., Ki, D., Rao, C. V., Jin, Y. S., & Guest, J. S. (2021). Sustainable Lactic Acid Production from Lignocellulosic Biomass. ACS Sustainable Chemistry and Engineering, 9(3), 1341-1351. https://doi.
[5] Bernardo, M. P., Coelho, L. F., Sass, D. C., & Contiero, J. (2016). I-(+)-Lactic acid production by Lactobacillus rhamnosus B103 from dairy industry waste. Brazilian journal of microbiology: [publication of the Brazilian Society for Microbiology], 47(3), 640-646. https://doi.
[6] De Man, J.C., Rogosa, M., & Sharpe, M.E. (1960). A medium for the cultivation of lactobacilli. Journal of Applied Bacteriology, 23, 130-135. https://doi.org/10.1111/j.1365-2672.1960.tb00188.x
[7] Guyot, J. P., Calderon, M., & Morlon-Guyot, J. (2000). Effect of pH control on lactic acid fermentation of starch by Lactobacillus manihotivorans LMG 18010T. Journal of applied microbiology, 88(1), 176-182. https://doi.org/10.1046/i.1365-2672.2000.00953.x.
[8] Ding, S. & Tan, T. (2006). I-lactic acid production by Lactobacillus casei fermentation using different fed-batch feeding strategies. Process Biochemistry, 41(6), 1451-1454. https://doi.
[9] Hetényi, K., Nemeth, A., & Sevella, B. (2011). Role of pH-regulation in lactic acid fermentation: Second steps in a process improvement. Chemical Engineering and Processing: Process Intensification, 50(3), 293-299. .
[10] Chou, L. S., & Weimer, B. (1999). Isolation and characterization of acid-and bile-tolerant isolates from strains of Lactobacillus acidophilus. Journal of Dairy Science, 82(1), 23-31. https://doi.org/10.3168/ids.S0022-0302(99).
[11] Pal, P., Sikder, J., Roy, S. & Giorno, L. (2009). Process intensification in lactic acid production: A review of membrane-based processes. Chemical Engineering and Processing: Process Intensification, 48(11), 1549-1559. .
[12] Madzingaidzo, L., Danner, H., & Braun, R. (2002). Process development and optimisation of lactic acid purification using electrodialysis. Journal of biotechnology, 96(3), 223-239. https://doi.org/10.1016/s0168-1656(02)
[13] Pleissner, D., Schneider, R., Venus, J., & Koch, T. (2017). Separation of lactic acid and recovery of salt-ions from fermentation broth. J. Chem. Technol. Biotechnol., 92, 504-511. https://doi.
[14] Olszewska-Widdrat, A., Alexandri, M., López-Gómez, J.P., Schneider, R., Mandl, M., Venus, J. (2019). Production and Purification of I-lactic Acid in Lab and Pilot Scales Using Sweet Sorghum Juice. Fermentation, 5(2), 36. https://doi.
[15] Papadopoulou, E., Cabrera, G., Mayuki, M. V., Reif, D., Ahmed, A., Tsapekos, P., Angelidaki, I. & Harasek, M. (2023). Separation of lactic acid from fermented residual resources using membrane technology. Journal of Environmental Chemical Engineering, 11(5) 110881. https://doi.
[16] Komesu, A., Martins, P. F., Lunelli, B. H., Rocha, J. O., Maciel Filho, R., & Wolf Maciel, M. R. (2015). The Effect of Evaporator Temperature on Lactic Acid Purity and Recovery by Short Path Evaporation. Separation Science and Technology, 50(10), 1548-1553. https://doi.
[17] Yu, J., Zeng, A., Yuan, X., Zhang, X., & Ju, J. (2015). Optimizing and Scale-Up Strategy of Molecular Distillation for the Purification of Lactic Acid from Fermentation Broth. Separation Science and Technology, 50(16), 2518-2524. https://doi.
[18] Komesu, A., Martins, P., Lunelli, B., Morita, A., Coutinho, P., Maciel Filho, R., & Wolf Maciel, M. (2013). Lactic Acid Purification by Hybrid Short Path Evaporation. Chemical Engineering Transactions, 32, 2017-2022. https://doi.
[19] Komesu, A., Martins, P., Lunelli, B., Oliveira, J., Maciel Filho, R., & Wolf Maciel, M. (2014). Evaluation of lactic acid purification from fermentation broth by hybrid short path evaporation using factorial experimental design. Separation and Purification Technology, 136, 233-240. https://doi.org/10.1016/j.seppur.2014.09.010

## Claims

1. A method of conducting electrodialysis (600) suitable for the conversion of sodium lactate into lactic acid, **characterised in that** a feed which is a sodium lactate solution with a concentration of 50 to 200 g/L of sodium lactate is introduced into a three-chamber electrodialyzer with a bipolar module made of cell pairs with a BPM-AM-CM membrane arrangement, and three products are obtained as a result of the method: a lactic acid solution, a sodium hydroxide solution and a depleted filtrate stream devoid of most sodium and lactate ions, and with the cathode and anode electrode solution being an aqueous sodium hydroxide solution.

2. The method of conducting electrodialysis (600) according to claim 1, **characterised in that** the initial conductivity of the electrode solution - sodium hydroxide is 20 mS/cm.

3. The method of conducting electrodialysis (600) according to claim 1, **characterised in that** the sodium lactate solution in the feed has a concentration of 50 - 130 g/L.

4. The method of conducting electrodialysis (600) according to any of the preceding claims, **characterised in that** the process is carried out at a limiting current density in the range of 50-500 A/m².

5. The method of conducting electrodialysis (600) according to any of the preceding claims, **characterised in that** the lactic acid solution obtained in the method is obtained by 9.9-99.9% conversion of lactate.

6. The method of conducting electrodialysis (600) according to any of the preceding claims, **characterised in that** the current efficiency of the unit electrodialysis process is in the range of 9.9-99.9%.

7. A method of obtaining lactic acid from fermentation broth using sucrose as the sole carbon source, comprising the following steps:
a. conducting bacterial fermentation (100) with pH control in the culture medium,
b. centrifugation (200) to separate the solid residues of the fermentation from the sodium lactate solution,
c. microfiltration in a membrane system (300),
d. membrane nanofiltration (400),
e. adsorption on activated carbon (500),
f. electrodialysis (600) carried out according to the method defined by any of the claims 1 to 6,
g. concentration (700).

8. The method of obtaining lactic acid according to claim 7, **characterised in that** the fermentation process a. (100) is carried out on a substrate containing molasses as the only sugar source, the pH of the fermentation process being regulated at 5.0 - 7.5, preferably 6.5, by dosing a sodium hydroxide solution, which is derived from the concentration (610) of the product of the electrodialysis process.

9. The method of obtaining lactic acid according to claim 7, **characterised in that** the solution after the adsorption step on activated carbon e. (500) has a colour of <100 APHA, preferably 10 APHA.

10. The method of obtaining lactic acid according to claim 7, **characterised in that** between the adsorption step on activated carbon e. (500) and electrodialysis step f. (600) an optional purification step on ion exchange beds (510) is carried out.

11. The method of obtaining lactic acid according to claim 7, **characterised in that** after the electrodialysis step f. (600) the concentration g. (700) of the obtained lactic acid solution is carried out to a concentration of 15 to 99%, preferably ≥76%.

12. A method of obtaining lactic acid from sodium lactate solution comprising the following steps:
a. membrane nanofiltration (400),
b. adsorption on activated carbon (500),
c. electrodialysis (600) carried out according to the method defined by any of the claims 1 to 6,
d. concentration (700).

13. The method of obtaining lactic acid according to claim 12, **characterised in that** after the electrodialysis step c. (600) the concentration d. (700) of the obtained lactic acid solution is carried out to a concentration of 15 to 99%, preferably ≥76%.

14. The method of obtaining lactic acid according to claims 12 or 13, **characterised in that** between the adsorption step on activated carbon b. (500) and the electrodialysis step c. (600) a purification step on ion exchange beds (510) is carried out.

15. The method of obtaining lactic acid according to claim 12, **characterised in that** the solution after the adsorption step on activated carbon b. (500) has a colour <100 APHA, preferably 9 APHA.
